# EUROPEAN PATENT SPECIFICATION

(11) **EP 3 976 261 B1**
(45) Date of publication and mention of the grant of the patent: **25.12.2024**
(21) Application number: 20812734.0
(22) Date of filing: 29.05.2020
(51) Int. Cl.: G01N 21/64, C12Q 1/6816, C12Q 1/686, B01L 3/00

(54) **METHOD FOR DETECTING A TARGET NUCLEIC ACID IN SAMPLE AND NUCLEIC ACID REACTION DETECTION DEVICE**
VERFAHREN ZUM NACHWEIS EINER ZIELNUKLEINSÄURE IN EINER PROBE UND VORRICHTUNG ZUM NACHWEIS EINER NUKLEINSÄUREREAKTION
PROCÉDÉ DE DÉTECTION D'UN ACIDE NUCLÉIQUE CIBLE DANS UN ÉCHANTILLON ET DISPOSITIF DE DÉTECTION D'UNE RÉACTION D'ACIDE NUCLÉIQUE

(30) Priority: 31.05.2019 KR 20190064472; 28.06.2019 KR 20190078284; 06.12.2019 KR 20190162094
(43) Date of publication of application: 06.04.2022
(73) Proprietor: Seegene, Inc., Seoul 05548 (KR)
(72) Inventor: KIM, Young Wook, Seoul 01724 (KR); LEE, Hye Jin, Anyang-si, Gyeonggi-do 13939 (KR); JEONG, Jun Hyeok, Seoul 05615 (KR)
(74) Representative: Brevalex
(86) International application number: PCT/KR2020/007040
(87) International publication number: WO 2020/242261

(56) References cited:
- US-A1- 2009 136 963
- US-A1- 2014 004 519
- US-A1- 2014 045 186
- US-A1- 2014 093 876
- US-A1- 2017 114 398
- US-A1- 2017 157 613

## Description

### Technical Field

The disclosure relates to a method for detecting a target nucleic acid in a sample using a nucleic acid reaction detection device.

### Background Art

Nucleic acid amplification reaction well known as polynucleotide chain reaction (PCR) includes repeated cycles of double-stranded DNA denaturation, annealing of the oligonucleotide primers to DNA templates, and extension/elongation of the primers with the DNA polymerase (Mullis et al, U.S. Patent Nos. 4,683,195, 4,683,202, and 4,800,159; Saiki et al, (1985) Science 230, 1350-1354). US2014/0045186 A1 discloses a system consisting of a microfluidic cartridge having multiple reaction chambers and an aperture plate with aligned apertures. The reaction chambers may be thermally controlled separately from one another or in groups. A moveable detector head with photodetector and light source pairs is configured to be positioned over each aperture allowing detection and analysis of reactions in the cartridge.

A typical PCR device (or machine) causes amplification in the reaction vessel positioned in the thermal block by varying the temperature of the thermal block according to a predetermined sequence. DNA denaturation is performed at about 95 °C, and primer annealing and elongation are performed at a lower temperature ranging from 55 °C to 75 °C. The temperature and reaction time for each step of DNA denaturation, annealing, and elongation need to be set to differ depending on, e.g., the sequence of oligonucleotides, e.g., the primers and probe used for analysis, the target nucleic acid for analysis, and each sample.

Real-time PCR is a PCR-based technique for detecting a target nucleic acid from a sample in real-time. To detect a particular target nucleic acid, a signal generator is used which radiates detectable fluorescent signals in proportion to the amount of the target nucleic acid upon PCR. To that end, an intercalator may be used which emits a fluorescent signal when combined with the dual-helix DNA, or oligonucleotides including a fluorophore and quencher molecules suppressing fluorescent emissions therefrom may be used. The fluorescent signal proportional to the amount of the target nucleic acid is detected in real-time at each cycle and is measured to obtain an amplification curve or amplification profile curve which displays the strength of the fluorescent signal detected per cycle.

The real-time PCR device is able to measure, in real-time, the signals while the nucleic acid reaction proceeds. Typically, the real-time PCR device illuminates the substrate with light and measures the resultant fluorescence, thereby detecting the nucleic acid in the reaction vessel. To that end, the real-time PCR device includes a light source for radiating an excitation light to the sample and a detector for receiving the light emitted from the sample.

Conventional nucleic acid reaction detection devices may analyze the nucleic acid reaction on multiple samples using a single device. However, the reaction proceeds under the same condition for all the samples and, since it is configured to radiate the same wavelength of light at the same time, only simultaneous analysis on the same mark for the plurality of samples is possible. Thus, analysis of various kinds of samples in small quantities takes an increased overall processing time and higher costs because each kind of sample needs to be analyzed separately.

To address such issues, a device has been developed which is equipped with a plurality of thermally independent thermal blocks and is able to perform nucleic acid reaction with different thermal protocols. However, this nucleic acid reaction detection device may simply amplify the nucleic acid without real-time optical measurement or, although performing nucleic acid reaction based on a different thermal protocol for eachthermal block thermally independent, analyzes the plurality of thermal blocks with one optical analyzing module. This device is unable to simultaneously measure optical signals of different wavelength bands from the samples individually received in the thermally-independent thermal blocks. Thus, the device cannot simultaneously analyze two or more nucleic acid reactions, for which the optical signal measurement time is independently determined, because the reaction is used based on the different thermal protocols.

Therefore, a need exists for a device capable of simultaneously performing different nucleic acid reactions in a single device and performing independent and real-time optical analysis and a method for detecting a nucleic acid using the same.

### Disclosure of Invention

### Technical Problem

The inventors have tried to develop a new method for detecting a target nucleic acid in a sample, for use in PCR devices comprising two or more reaction areas thermally independent from each other such that two or more nucleic acid reactions based on distinct protocols are capable of being performed simultaneously in a single device. As a result, the inventors have found that the target nucleic acid in the sample included in each sample set may be detected by positioning a first sample set in a first reaction area and a second sample set in a second reaction area in a nucleic acid reaction detection device, which comprises a sample reaction module comprising the first reaction area and the second reaction area thermally independent from each other, an optical module capable of detecting nucleic acid reaction in samples in the first reaction area and the second reaction area, and a controller, and in which the temperatures of the first reaction area and the second reaction area are controlled according to protocols independent from each other, performing nucleic acid reaction, synchronously measuring optical signals from the first sample set and the second sample set, wherein the wavelength bands of the optical signals from the first sample set and the second sample set are different from each other and analyzing the measured optical signals.

Thus, a purpose of the present disclosure is to provide a method for detecting a target nucleic acid in a sample.

Another purpose of the present disclosure is to provide a nucleic acid reaction detection device that may synchronously measure an optical signal of a first wavelength band generated from the sample in the first reaction area and an optical signal of a second wavelength band (which differs from the first wavelength band) generated from the sample in the second reaction area.

The other objectives and advantages of the disclosure will be apparent from the embodiments and claims described below in detail with reference to the accompanying drawings.

### Solution to Problem

To achieve the foregoing objects, according to one aspect of the invention, there is provided a method for detecting a target nucleic acid in a sample as defined in claim 1.

To achieve the foregoing objects, according to another aspect of the invention, there is provided a nucleic acid reaction detection device as defined in claim 9. module is configured to synchronously measure an optical signal of a first wavelength band from a sample in the first reaction area and an optical signal of a second wavelength band from a sample in the second reaction area, wherein the second wavelength band is different from the first wavelength band, and wherein the controller is configured to control temperatures of the first reaction area and the second reaction area independently and control optical signal measurement of a sample in the first reaction area and optical signal measurement of a sample in the second reaction area independently.

### Advantageous Effects of Invention

According to the disclosure, the method and device may simultaneously perform two or more nucleic acid reactions which are carried out with different protocols and independently measure emitted optical signals, thereby detecting the target nucleic acid. The method and device may thus analyze various kinds of samples in small quantities, providing advantages in light of sample analysis time and costs.

Where the optical module measures optical signals while being moved by a moving means, the measurement takes long and the measurement time is varied between the individual samples. However, the device and method according to the disclosure are able to synchronously measure optical signals of different wavelength bands generated from a plurality of samples included in the sample set where nucleic acid reaction has been performed in different reaction areas and may thus detect the target nucleic acid in a precise and efficient manner as compared with conventional analyzers which may perform optical signal measurement only sequentially.

The method and device according to the disclosure may detect two or more target nucleic acids in one sample. Therefore, the method and device according to the disclosure enable multiplex target nucleic acid detection from two or more sample sets for detecting different target nucleic acids.

### Brief Description of Drawings

Fig. 1 is a view illustrating a nucleic acid reaction detection device according to an embodiment of the disclosure;
Fig. 2 is a view illustrating a structure of a nucleic acid reaction detection device with a separate light source for each reaction area, according to an embodiment of the disclosure;
Fig. 3 is a view illustrating a structure of a nucleic acid reaction detection device with a light source shared by a plurality of reaction areas, according to an embodiment of the disclosure;
Fig. 4 is a view illustrating a structure in which a separate light source is provided for each reaction area according to an embodiment of the disclosure;
Fig. 5 is a view illustrating a structure in which a light source is shared by two or more reaction areas according to an embodiment of the disclosure;
Fig. 6 is a view illustrating a sample reaction module and an optical module using a light source comprising a plurality of light emitting elements in a nucleic acid reaction detection device according to an embodiment of the disclosure;
Fig. 7 is a view illustrating an optical path of a nucleic acid reaction detection device according to an embodiment of the disclosure; and
Fig. 8 is a view illustrating a structure in which a controller is electrically connected with another component of a nucleic acid reaction detection device according to an embodiment of the disclosure.

### Mode for the Invention

The configuration and effects of the present invention are now described in further detail in connection with embodiments thereof. The embodiments are provided merely to specifically describe the present invention. The scope of the invention is not limited by these embodiments but by the appended claims.

### I. Detection of target nucleic acid in sample

According to the invention, there is provided a method for detecting a target nucleic acid in a sample, comprising, *inter alia:*
(a) positioning a first sample set in a first reaction area of a nucleic acid reaction detection device and a second sample set in a second reaction area of the nucleic acid reaction detection device and performing a nucleic acid reaction; wherein the nucleic acid reaction detection device comprises a sample reaction module comprising the first reaction area and the second reaction area thermally independent from each other, an optical module for detecting nucleic acid reaction in the first reaction area and the second reaction area, and a controller; wherein temperatures of the first reaction area and the second reaction area are controlled according to different protocols;
(b) measuring optical signals from the first sample set and the second sample set; wherein the optical signals from the first sample set and the second sample set are measured synchronously; wherein the wavelength bands of the optical signals synchronously measured from the first sample set and the second sample set are different from each other; and
(c) detecting a target nucleic acid in a sample included in the first sample set and the second sample set by analyzing the measured optical signals.

The inventors have tried to develop a new method for detecting a target nucleic acid in a sample, for use in PCR devices comprising two or more reaction areas thermally independent from each other such that two or more nucleic acid reactions based on distinct protocols are capable of being performed simultaneously in a single device. As a result, the inventors have found that the target nucleic acid in the sample included in each sample set may be detected by positioning a first sample set in a first reaction area and a second sample set in a second reaction area in a nucleic acid reaction detection device, which comprises a sample reaction module comprising the first reaction area and the second reaction area thermally independent from each other, an optical module capable of detecting nucleic acid reaction in samples in the first reaction area and the second reaction area, and a controller, and in which the temperatures of the first reaction area and the second reaction area are controlled according to protocols independent from each other, performing nucleic acid reaction, synchronously measuring optical signals from the first sample set and the second sample set, and analyzing the measured optical signals.

Each step of the method is described below in detail:

### Step (a): nucleic acid reaction

According to the disclosure, the first sample set and the second sample set are positioned in the first reaction area and the second reaction area, respectively, of the nucleic acid reaction detection device, and nucleic acid reaction is performed.

According to an embodiment, the nucleic acid reaction detection device refers to a device that detects the target nucleic acid using nucleic acid reaction. In particular, the nucleic acid reaction detection device is a device that comprises a sample reaction module comprising the first reaction area and the second reaction area thermally independent from each other, an optical module for detecting nucleic acid reaction of samples in the first reaction area and the second reaction area, and a controller, and in which the temperatures of the first reaction area and the second reaction area may be controlled according to different protocols. The nucleic acid reaction detection device is described in more detail below in Section II.

As used herein, the term "sample" may encompass biological samples (e.g., cells, tissues, or fluids from biological sources) and non-biological samples (e.g., foods, water, and soil). The biological samples include virus, germs, tissues, cells, blood (e.g., whole blood, plasma, and serum), lymph, bone marrow fluid, saliva, sputum, swab, aspiration, milk, urine, stool, ocular humor, semen, brain extracts, spinal fluid, joint fluid, thymus fluid, bronchoalveolar lavage fluid, ascites, and amniotic fluid. The sample may undergo a nucleic acid extraction process. Where the extracted nucleic acid is RNA, reverse transcription may be added to synthesize cDNA from the extracted RNA (Joseph Sambrook, et al., Molecular Cloning, A Laboratory Manual, Cold Spring Harbor Laboratory Press, Cold Spring Harbor, N.Y.(2001)).

The nucleic acid reaction means a series of physical or chemical reactions that generate a signal dependent upon the presence or absence of a nucleic acid with a specific sequence in the sample or the amount thereof. The nucleic acid reaction may be a reaction that includes a hybridization of the nucleic acid with the specific sequence in the sample and other nucleic acid or material, copying, cutting, or decomposition of the nucleic acid with the specific sequence in the sample.

According to an embodiment of the disclosure, the nucleic acid reaction may be a signal-generating process that may generate a signal, dependent upon the presence or absence of target nucleic acid in the sample or the amount thereof. The signal-generating process may be a genetic analysis process, such as PCR, real-time PCR, or microarray. According to an embodiment of the disclosure, the nucleic acid reaction may be PCR or real-time PCR.

According to an embodiment of the disclosure, the signal-generating process may be an amplification reaction of signal. The amplification reaction means a reaction to increase or decrease signals. According to an embodiment of the disclosure, the amplification reaction means increasing (or amplifying) signals, relying on the presence of the target nucleic acid using a signal-generation means. The amplification reaction may, or may not, be accompanied by amplification of the target nucleic acid. Thus, according to an embodiment of the disclosure, the nucleic acid reaction may be performed along with, or without, amplification of target nucleic acid molecules. Specifically, according to the disclosure, the nucleic acid reaction may be a signal amplification reaction that is performed accompanied by amplification of the target analyte.

The signal-generating process is accompanied by a signal change. As used herein, the term "signal" means a measurable output.

The signal change may serve as an indicator qualitatively or quantitatively indicating the presence/absence of the target nucleic acid or the amount thereof. Examples of the indicator include the strength of fluorescence, the strength of luminescence, the strength of chemiluminescence, the strength of bioluminescence, and the strength of phosphorescence. The indicator most widely used is the strength of fluorescence. The signal change may include a signal decrease as well as a signal increase.

As used herein, 'signal' refers to an optical signal. The optical signal may be luminescence, phosphorescence, chemiluminescence, fluorescence, polarized fluorescence, or other colored signal.

To generate the optical signal, a signal-generating means may be included in the sample. A wide variety of the signal-generating means have been known to one of skill in the art. The signal-generating means comprises a label itself or a label-attached oligonucleotide. The label may include a fluorescent label, a luminescent label, a chemiluminescent label, an electrochemical label, and a metal label. The label per se like an intercalating dye may serve as signal-generating means. Alternatively, a single label or interactive dual label including a donor molecule and an acceptor molecule may be used as signal-generating means in the form of linkage to at least one oligonucleotide. The signal-generating means may comprise additional components for generating signals such as an enzyme with nucleolytic activity (e.g., 5'-nucleases and 3'-nucleases).

According to an embodiment of the disclosure, more specifically, the label used for the signal-generating means may include a single fluorescent label or interactive dual label (including, e.g., a fluorescence reporter molecule and a quencher molecule) including a donor molecule and an acceptor molecule. The fluorescent label or fluorescent reporter molecule comprises, but is not limited to, FAM^{™}, TET^{™}, VIC^{™}, JOE^{™}, HEX^{™}, CY3, TAMRA^{™}, ROX^{™}, Texas Red, CY5, CY5.5, and Quasar 705.

There are known various methods of generating an optical signal to indicate the presence of the target nucleic acid using nucleic acid reaction. Representative examples include: TaqMan^{™} probe method (U.S. Patent No. 5,210,015), molecule beacon method (Tyagi et al., Nature Biotechnology v.14 MARCH 1996), Scorpion method (Whitcombe et al., Nature Biotechnology 17:804-807(1999)), Sunrise or Am-plifluor method (Nazarenko et al., 2516-2521 Nucleic Acids Research, 25(12):2516-2521(1997), and U.S. Patent No. 6,117,635), Lux method (U.S. Patent No. 7,537,886), CPT(Duck P, et al. Biotechniques, 9:142-148(1990)), LNA method (U.S. Patent No. 6,977,295), Plexor method (Sherrill CB, et al., Journal of the American Chemical Society, 126:4550-4556(2004)), Hybeacons^{™} (D. J. French, et al., Molecular and Cellular Probes (2001) 13, 363-374 and U.S. Patent No. 7,348,141), Dual-labeled, self-quenched probe; U.S. Patent No. 5,876,930), hybridization probe (Bernard PS, et al., Clin Chem 2000, 46, 147-148), PTOCE(PTO cleavage and extension) method (WO 2012/096523), PCE-SH(PTO Cleavage and Extension-Dependent Signaling Oligonucleotide Hybridization) method (WO 2013/115442), PCE-NH(PTO Cleavage and Extension-Dependent Non-Hybridization) method (PCT/KR2013/012312) and CER method (WO 2011/037306).

According to an embodiment, the nucleic acid reaction detection device may be a device that performs nucleic acid amplification reaction to amplify the nucleic acid with a specific nucleic sequence. The target nucleic acid may be amplified by various methods. For example, methods for amplifying the target nucleic acid include the polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA)) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)) and Q-beta Replicase (Lizardi et al., BiolTechnology 6:1197 (1988)).

In particular, according to an embodiment, the nucleic acid reaction detection device may be a device that performs nucleic acid amplification reaction, accompanied by a temperature change. For example, to amplify deoxyribonucleic acid (DNA) with a specific nucleic sequence, the nucleic acid amplification reaction may include a denaturing step, an annealing step, and an extension (or amplification) step.

According to an embodiment, the nucleic acid reaction detection device may be a device that performs the target nucleic acid amplification reaction, accompanied by a temperature change, and the reaction of generating an optical signal, dependent on the presence of the target nucleic acid and detects a generated optical signal.

The nucleic acid reaction detection device comprises a sample reaction module. The sample reaction module is a module that receives a sample, the target for analysis, and performs nucleic acid reaction. The sample reaction module comprises a reaction area for receiving the sample or a reaction vessel containing the sample. A plurality of samples may be received in the sample reaction module. The sample reaction module according to the disclosure comprises a first reaction area and a second reaction area thermally independent from each other.

According to an embodiment of the disclosure, the sample reaction module may comprise other additional reaction area(s) than the first reaction area and the second reaction area. For example, the sample reaction module may further comprise a third reaction area and a fourth reaction area. The reaction areas each may be controlled thermally independently. The reaction areas being controlled thermally independently may mean that the temperature of each reaction area may be individually controlled. For example, while the first reaction area remains at 94 °C at a first time, the second reaction area may be cooled down from 94 °C to 60 °C or heated up from 60 °C to 75 °C or may remain at a specific temperature. By so doing, a separate nucleic acid reaction may be performed in each reaction area of the nucleic acid reaction detection device. The number of reaction areas included in the sample reaction module may range from 2 to 24, but is not limited thereto. For example, the number of reaction areas comprised in the sample reaction module may be 2, 3, 4, 5, 6, 8, 10, 12, 16, 20, or 24. According to an embodiment of the disclosure, the reaction areas are thermally independent from each other. In other words, heat does not transfer from one reaction area to another. For example, a thermal insulation material or an air gap may be provided between the reaction areas.

In the nucleic acid reaction step, the temperatures of the first reaction area and the second reaction area are controlled according to protocols independent from each other.

As used herein, the protocol means a set of instructions to perform a unit operation. The unit operation includes the operation of controlling temperature over time and the operation of measuring signal. The protocol comprises information regarding the order and timings of performing various unit operations in the protocol.

The method according to the disclosure is characterized by allowing the first sample set and the second sample set to react according to protocols independent from each other. Sample set refers to a set of samples including one or more samples. For the samples included in one sample set, nucleic acid reaction and detection are performed according to the same protocol. According to an embodiment of the disclosure, the first sample set may comprise a plurality of samples.

The first sample set and the second sample set are samples for detecting different target nucleic acids from each other. Thus, the optimal protocols for the first sample set and second sample set differ from each other for efficient nucleic acid reaction and detection. The sample reaction module of the present disclosure may control temperature independently for each reaction area and, thus, for the first sample set and second sample set, reaction may be performed according to protocols independent from each other.

### Step (b): measurement of optical signal

In the step of optical signal measurement, optical signals from the first sample set and the second sample set are measured. Optical signal measurement means measuring an optical signal that is generated depending on the presence of the target nucleic acid in the sample or the amount thereof.

In the method according to the disclosure, the nucleic acid reaction may be repeated several times. According to an embodiment of the disclosure, optical signal measurement may be performed once after the plurality of nucleic acid reactions all have been finished. Selectively, according to an embodiment of the disclosure, the optical signal measurement may be performed several times between the plurality of nucleic acid reactions. Preferably, the optical signal measurement may be performed for each nucleic acid reactions of the plurality of nucleic acid reactions.

Further, since nucleic acid reaction is performed on the first sample set and the second sample set according to independent protocols, the times for measuring the optical signals for the first sample set and the second sample set are determined independently from each other.

Specifically, according to the invention, the measurement step comprises the step of synchronously measuring optical signals from the first sample set and the second sample set. Or, the measurement step of the disclosure may further comprise the step of measuring an optical signal from one sample set of the first sample set and the second sample set without measuring an optical signal for the other sample set of the first sample set and the second sample set. The measurement step of the disclosure, which comprises a number of measurements of optical signals, generally comprises both of the measurement steps disclose above. The wavelength bands of the optical signals measured from the first sample set and the second sample set may be identical to or different from each other.

The conventional target nucleic acid detector, which has thermal blocks thermally independent from each other and may thus simultaneously perform different nucleic acid reactions, lacks an optical module capable of real-time optical measurement or is configured to simultaneously measure the thermal blocks at the same wavelength. The device with such a structure may only adopt the method of collecting the sample after nucleic acid reaction is finished and performing analysis separately or the method of simultaneously measuring optical signals of the same wavelength band on all the samples where nucleic acid reaction proceeds in the device. Such a measurement method is not suitable for real-time analysis of different nucleic acid reactions which are performed by independent protocols in a single device.

The target nucleic acid detection method according to the disclosure comprises the step of synchronously measuring an optical signal measured from the first sample set and an optical signal measured from the second sample set, which are of different wavelength bands. According to an embodiment of the disclosure, the target nucleic acid detection method according to the disclosure comprises the measurement step of synchronously measuring optical signals from the first sample set and the second sample set. The optical signal measured from the first sample set and the optical signal measured from the second sample set have different wavelength bands. The optical signals of different wavelength bands mean optical signals generated from different labels. Thus, the target nucleic acid detection method according to the disclosure may comprise the step of synchronously detecting different labels from the first sample set and the second sample set.

The synchronous measurement may encompass the case where optical signals for two reaction areas are measured at the same time. The synchronous measurement may also encompass the case where although the times of measurement of optical signals for two reaction areas do not simply overlap each other, the optical signal for one reaction area is measured and, then, the optical signal for the other reaction area is measured without any physical component, e.g., a light source or filter for the device. The synchronous measurement may also encompass the case where measurement data of the optical signals for two reaction areas is treated as obtained in substantially the same time period.

According to the invention, the measuring step comprises the step of synchronously measuring the optical signals from the first sample set and the second sample set, wherein the wavelength bands of the optical signals synchronously measured from the first sample set and the second sample set are different from each other. The measuring step may further comprise the step of measuring an optical signal from one of the first sample set and the second sample set without measuring an optical signal from the other of the first sample set and the second sample set.

According to an embodiment of the disclosure, in the target nucleic acid detection method according to the disclosure, the measuring step may further comprise the step of measuring the optical signal from the first sample set without measuring the optical signal for the second sample set. According to an embodiment of the disclosure, in the target nucleic acid detection method according to the disclosure, the measuring step may further comprise the step of measuring the optical signal from the second sample set without measuring the optical signal for the first sample set.

When real-time PCR is performed on the first sample set and the second sample set according to separate protocols in the single device, the periods of measurement of the optical signals for the two sample sets differ from each other. In this case, the single device may synchronously measure optical signals of different wavelength bands from the first sample set and the second sample set in some cycle and, in other cycle, may measure the optical signal from one sample set of the first sample set and the second sample set without measuring the optical signal for the other sample set of the first sample set and the second sample set.

The wavelength band of the measured optical signal may include the whole or part of the wavelength band of the emission light generated from the used label. The wavelength band of the measured optical signal may include the whole or part of the wavelength band of the emission light generated from one or more label selected from the group consisting of, but not limited to, FAM^{™}, TET^{™}, VIC^{™}, JOE^{™}, HEX^{™}, CY3, TAMRA^{™}, ROX^{™}, Texas Red, CY5, CY5.5, and Quasar 705.

The method according to the disclosure may apply to real-time multiplex nucleic acid detection methods. Multiplex nucleic acid detection means distinctively detecting, in real-time, two or more target nucleic acids in one sample. To assay optically real-time multiplex nucleic acid detection, there is needed the step of measuring optical signals of two or more wavelength bands respectively corresponding to the target nucleic acids in one sample. Thus, according to an embodiment of the disclosure, the measurement of an optical signal for the first sample set may be performed by sequentially measuring optical signals of two or more different wavelength bands for the first sample set. The number of the different wavelength bands may be two or more or may be three or more, or may be seven or less or may be six or less.

According to an embodiment of the disclosure, the first sample set may comprise a plurality of samples. Measuring an optical signal from the first sample set positioned in the first reaction area may be simultaneously measuring optical signals from two or more samples included in the first sample set. The number of the samples included in the first sample set, although not specifically limited, may be, e.g., 1 or 2 or more or may be 1563, 1000, 800, 500, 400, 384, 100, 96, 32 or less.

According to an embodiment of the disclosure, the second sample set may comprise a plurality of samples. Measuring an optical signal from the second sample set positioned in the second reaction area may be simultaneously measuring optical signals from two or more samples included in the second sample set. The number of the samples included in the second sample set, although not specifically limited, may be, e.g., 1 or 2 or more or may be 1563, 1000, 800, 500, 400, 384, 100, 96, 32 or less.

According to an embodiment of the disclosure, the nucleic acid reaction detection device comprises an optical module capable of detecting nucleic acid reaction in the samples in the first reaction area and second reaction area. According to an embodiment of the disclosure, the optical signal measurement is performed by the optical module of the nucleic acid reaction detection device. The optical module comprises a light source for illuminating the sample in the reaction area with an excitation light and a detector for sensing an emission light received from the sample in the reaction area. The detector may be, e.g., a CCD (Charge Coupled device), CMOS (Complementary Metal Oxide Semiconductor field effect transistor) or photodiode. The light radiated from the light source may also be referred to as an excitation light, and the light emitted from the label excited by the excitation light may also be referred to as an emission light.

To synchronously measure the optical signal from the first sample set and the optical signal from the second sample set, which are of different wavelength bands, different wavelength bands of light need to be synchronously radiated to the first reaction area where the first sample set is positioned and the second reaction area where the second sample set is positioned, and the emission lights generated from the first sample set and the second sample set need to be synchronously sensed.

Thus, the light source of the optical module is characterized by being able to synchronously radiate different lights to the first reaction area where the first sample set is positioned and the second reaction area where the second sample set is positioned. Further, the detector of the optical module is characterized by being able to synchronously sense the emission lights generated from the first sample set and the second sample set.

According to an embodiment of the disclosure, the optical module comprises a plurality of light sources. At least two of the plurality of light sources may produce excitation lights of different wavelength bands from each other. For example, the optical module may comprise a first light source and a second light source. The wavelength bands of the lights radiated from the first light source and the second light source may differ from each other. Alternatively, the optical module may comprise a first light source to a fourth light source. The wavelength bands of the lights radiated from the first light source and the second light source may differ from each other. Alternatively, the optical module may comprise a first light source to a fourth light source. The wavelength bands of the lights radiated from the first light source to the fourth light source may differ from each other. The number of the light sources comprised in the optical module is not particularly limited but, as an example, may be two or more or may be 100, 50, 40, 30, 20, 10 or less.

As such, when the optical module of the nucleic acid reaction detection device used for the method according to the disclosure comprises a plurality of light sources, and at least two of the plurality of light sources are configured to produce excitation lights of different wavelength bands from each other, it may be possible to synchronously measure optical signals of different wavelength bands from the first sample set and the second sample set.

Meanwhile, according to an embodiment of the disclosure, the optical module may comprise a plurality of detectors. Each of the plurality of detectors may detect light emitted from a sample in a predetermined and fixed area of the sample reaction module.

The detector senses the emission light generated from the sample. The detector may be, e.g., a CCD (Charge Coupled device), CMOS (Complementary Metal Oxide Semiconductor field effect transistor) or photodiode.

In the method according to the disclosure, there may be provided a plurality of detectors. When a plurality of detectors are used, the number of samples sensed by each detector may be reduced, so that more accurate detection is possible. The number of the detectors, although not specifically limited, may be, e.g., 2, 3, 4, or more or may be 100, 90, 80, 70, 60, 50, 40, 30, 20, 10, 9, 8, 7, 6 or less.

According to an embodiment of the disclosure, the optical module comprises a plurality of detectors independently controlled, and one or more different detectors among the plurality of detectors may be assigned to each of the first reaction area and the second reaction area. Optical signals may be measured from the first sample set and second sample set by the one or more different detectors assigned.

The plurality of detectors each may sense an emission light generated from one reaction area. In the method according to the disclosure, one or more detectors may be assigned to each reaction area, and the two or more detectors may sense emission lights, with each reaction area segmented. For example, where the nucleic acid reaction detection device used for the method according to the disclosure comprises two reaction areas thermally independent from each other and comprises four detectors, a first detector and a second detector may sense the emission light generated from the sample positioned in the first reaction area, and a third detector and a fourth detector may sense the emission light generated from the sample positioned in the second reaction area.

In the method according to the disclosure, each detector may be a detector that senses the emission light generated from the sample in a predetermined and fixed detection area of the sample reaction module. The detection area means an area where one detector detects an emission light in the sample reaction module. When the plurality of detectors each sense the emission light generated from the sample positioned in the predetermined detection area, this means that the detection area where each detector performs detection in the sample reaction module is previously determined. The samples positioned in the same detection area in the sample reaction module may be detected by the same detector, and the samples positioned in different detection areas may be detected by different detectors. For example, a first detector may detect only emission lights generated from a first detection area, which is a fixed area in the sample reaction module but may not detect emission lights generated from other area.

The nucleic acid reaction detection device comprises a controller. The controller may be electrically connected with the sample reaction module and the optical module. The controller controls the temperatures of the first reaction area and the second reaction area independently and controls optical signal measurement of the sample in the first reaction area and optical signal measurement of the sample in the second reaction area independently. There may be provided one or more controllers.

According to an embodiment of the disclosure, the controller is characterized by operating the nucleic acid reaction detection device to perform an operation comprising at least one of the steps of:
(i) measuring an optical signal for one sample set of the first sample set and the second sample set without measuring an optical signal for the other sample set and (ii) synchronously measuring the optical signals from the first sample set and the second sample set, wherein the wavelength bands of the optical signals synchronously measured from the first sample set and the second sample set are different from each other.

Reaction is performed on the sample positioned in the first reaction area and the sample positioned in the second reaction area by protocols independent from each other, and the first reaction area and the second reaction area may individually or simultaneously reach the light detection time. When it becomes time to detecting optical signal only for one of the first reaction area and the second reaction area, the controller operates to perform the operation of measuring the optical signal for the sample in one reaction area of the first reaction area and the second reaction area without measuring the optical signal for the sample in the other reaction area. Further, when it becomes time to detecting optical signals for both the first reaction area and the second reaction area, the controller operates to perform the operation of measuring the optical signal for the sample in each reaction area. At this time, the wavelength bands of the optical signals measured from the first reaction area and the second reaction area may differ from each other.

### Step (c): detection of target nucleic acid

According to the disclosure, the measured optical signals are analyzed, detecting the target nucleic acid in the sample included in the first sample set and the second sample set.

The detection of the target nucleic acid means quantitatively or qualitatively measuring the target nucleic acid in the sample using the optical signals measured from the sample. The quantitative or qualitative detection may be the detection of the presence or absence of the target nucleic acid in the sample or the detection of the amount of the target nucleic acid in the sample.

The detection may be performed by a known detection method.

The detection may be performed by a method of determining that the target nucleic acid corresponding to the measured optical signal is present in the sample when the value of the measured optical signal is a predetermined threshold or more. Alternatively, the detection may be performed by a method of calculating the variation in the fluorescent signal, which is increased as the nucleic acid reaction is repeated and determining whether there is the target nucleic acid based on the calculated variation. Alternatively, the detection may be performed by a method of obtaining a standard curve using a reference sample including the target nucleic acid in various concentrations, comparing the standard curve with the optical signal measured from each sample, and calculating the initial amount of the target nucleic acid included in each sample. Alternatively, the detection may be the detection of the target nucleic acid based on the optical signals obtained by melting analysis between the target nucleic acid and the signal-generating means in each of the nucleic acid reaction repeated.

### II. nucleic acid reaction detection device

According to another embodiment of the disclosure, there is provided a nucleic acid reaction detection device.

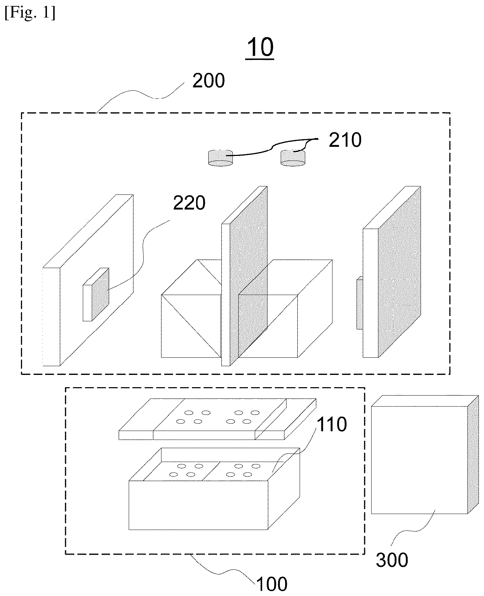

Fig. 1 is a view illustrating a nucleic acid reaction detection device 10 according to an embodiment of the disclosure.

Referring to Fig. 1, the nucleic acid reaction detection device 10 according to the disclosure comprises a sample reaction module 100 comprising a first reaction area and a second reaction area thermally independent from each other, an optical module 200 capable of detecting nucleic acid reaction of all samples in the sample reaction module, and one or more controllers 300 electrically connected with the sample reaction module and the optical module. In the nucleic acid reaction detection device according to the disclosure, the optical module is configured to be able to synchronously measure an optical signal of a first wavelength band from a sample in the first reaction area and an optical signal of a second wavelength band from a sample in the second reaction area, wherein the second wavelength band differs from the first wavelength band. The controller is configured to control the temperatures of the first reaction area and the second reaction area independently and control optical signal measurement of the sample in the first reaction area and optical signal measurement of the sample in the second reaction area independently.

According to an embodiment of the disclosure, the nucleic acid reaction detection device 10 comprises the sample reaction module 100. The sample reaction module 100 is a module that receives samples which are to be analyzed and performs nucleic acid reaction.

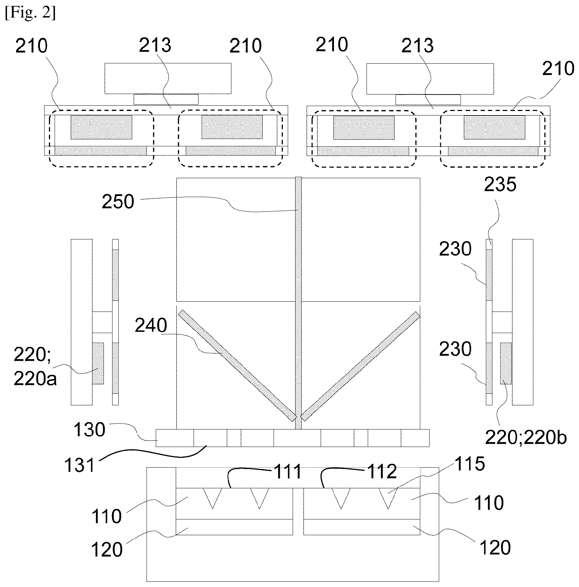

Referring to Fig. 2, the sample reaction module 100 comprises a first reaction area 111 and a second reaction area 112. Each reaction area is thermally independent from another. A thermal insulation material or air gap may be provided between the first reaction area and the second reaction area to prevent heat transfer. According to an embodiment of the disclosure, the sample reaction module 100 comprises the first reaction area 111 and the second reaction area 112 thermally independent from each other. The sample reaction module 100 may comprise reaction areas 110 and heat transfer modules 120.

According to an embodiment of the disclosure, the sample reaction module 100 may be configured so that each of the first reaction area 111 and the second reaction area 112 is able to receive at least two or more samples. To that end, the sample reaction module 100 comprises the reaction area 110 for receiving the sample or a reaction vessel including the sample. The reaction area 110 may comprise a plurality of receiving spaces 115. The receiving space 115 for receiving the sample may be a well for receiving the reaction vessel. Each reaction area may receive at least two or more samples using the plurality of receiving spaces 115. Although not particularly limited, the number of the receiving spaces 115 included in each reaction area may be 2 to 96, 2 to 84, 2 to 72, 2 to 64, 2 to 56, 2 to 48, 2 to 40, or 2 to 32. The sample reaction module 100 adjusts the temperature of the received samples and performs nucleic acid reaction. Thus, the reaction area 110 may be formed of a metal or metal alloy with superior thermal conductivity. The reaction area 110 may be, e.g., a thermal block. The thermal block has a volume and is formed of a metal with superior thermal conductivity. The thermal block may have a well for receiving samples. A heat transfer module, e.g., a Peltier element, may contact one surface of the thermal block to adjust the temperature.

Another example of the reaction area 110 is a heating plate. The heating plate may be formed of a plate for receiving samples and a thin metal sheet attached to the plate. The heating plate may be operated in such a manner that the plate is heated by applying electric current to the thin metal sheet.

Another example of the reaction area 110 may be formed of one or more chips or cartridges. An example of the cartridge is a fluid cartridge comprising a flow channel.

The sample reaction module may further comprise the heat transfer module 120 for supplying heat to the reaction area 110 or absorbing heat from the reaction area 110. The heat transfer module 120 may switch the reaction area 110 into two or more temperatures. The heat transfer module 120 may be a device capable of heating and cooling both. The heat transfer module 120 may separately comprise a heat supplying means for supplying heat and a heat absorbing means for absorbing heat. According to an embodiment of the disclosure, the heat transfer module 120 may be a Peltier element capable of both heat supply and heat absorption to/from the reaction area 110 and positioned in the form of a plate on the bottom of the reaction area 110.

As shown in Fig. 2, in the nucleic acid reaction detection device according to the disclosure, the first reaction area and the second reaction area may be reaction areas 110 physically separated from each other. The nucleic acid reaction detection device according to the disclosure may comprise heat transfer modules 120 physically separated from each other to change the temperatures of the first reaction area and the second reaction area independently. Thus, in the nucleic acid reaction detection device according to the disclosure, the first reaction area 111 and the second reaction area 112 may be controlled thermally independently from each other.

The reaction areas 111 and 112 may be controlled thermally independently from each other. The reaction areas being controlled thermally independently may mean that the temperature of each reaction area may be individually controlled. For example, while the first reaction area remains at 94 °C at a first time, the second reaction area may be cooled down from 94 °C to 60 °C or heated up from 60 °C to 75 °C or may remain at a specific temperature. By so doing, a separate nucleic acid reaction may be performed in each reaction area of the nucleic acid reaction detection device. The number of reaction areas included in the sample reaction module may range from 2 to 24, but is not limited thereto. For example, the number of reaction areas included in the sample reaction module may be 2, 3, 4, 5, 6, 8, 10, 12, 16, 20, or 24. According to an embodiment, the nucleic acid reaction detection device may comprise two or more reaction areas, four or more reaction areas, six or more reaction areas, or eight or more reaction areas. According to an embodiment, the nucleic acid reaction detection device may comprise 24 or less reaction areas, 20 or less reaction areas, 16 or less reaction areas, or 12 or less reaction areas. According to an embodiment of the disclosure, the sample reaction module may comprise other additional reaction area(s) than the first reaction area and the second reaction area. For example, the sample reaction module may further comprise a third reaction area and a fourth reaction area. The reaction areas each may be controlled thermally independently.

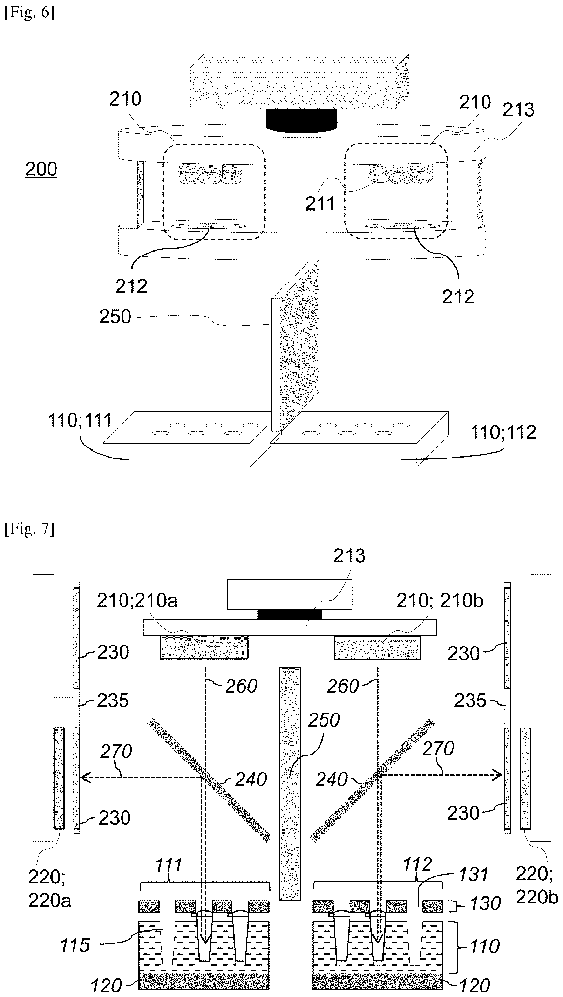

Meanwhile, the sample reaction module 100 according to the disclosure may further comprise a pressure lid 130. Referring to Fig. 7, the pressure lid 130 provides pressure to the reaction vessels containing samples positioned on the reaction area 110. The pressure lid 130 may contact the covers of the reaction vessels and press the covers of the reaction vessels, providing pressure to the reaction vessels. The pressure lid 130 may maintain a high temperature. For example, the pressure lid 130 may comprise a heat plate (not shown) to maintain a temperature of 105 °C. The pressure lid 130 comprises a plurality of holes 131. The holes 131 of the pressure lid 130 are formed in the positions corresponding to the reaction vessels containing samples, positioned on the reaction area 110.

Referring to Fig. 1, the nucleic acid reaction detection device according to the disclosure comprises the optical module 200 capable of detecting nucleic acid reaction of the samples in the sample reaction module.

The optical module 200 radiates excitation lights to the samples in the sample reaction module 100 and senses the emission lights generated by the excitation lights, thereby detecting the nucleic acid reaction in the sample.

According to an embodiment of the disclosure, the optical module 200 may comprise a light source 210 for illuminating the sample in the reaction area with an excitation light and a detector 220 for sensing an emission light received from the sample in the reaction area.

The light source 210 illuminates the sample in the reaction area with an excitation light. The excitation light refers to the light generated from the light source 210 and reaching the sample positioned in the sample receiving space 115. The label in the sample may be excited by the excitation light. The excited label may emit an optical signal, e.g., fluorescent light. The light radiated from the light source 210 may also be referred to as an excitation light, and the light emitted from the label excited by the excitation light may also be referred to as an emission light.

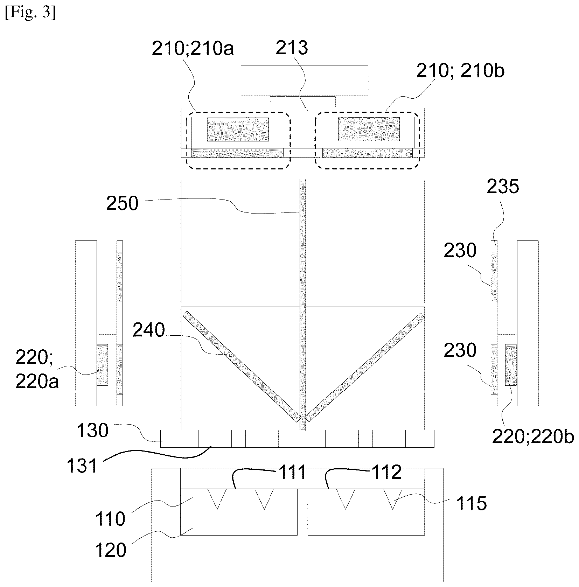

Referring to Fig. 3, there may be provided a plurality of light sources 210. In such a case, the optical module 200 is configured so that, when a first light source 210a radiates light to the first reaction area 111, a second light source 210b may radiate light of a different wavelength band from that of the first light source 210a to the second reaction area 112. In this case, the optical module 200 may synchronously measure the optical signal of the first wavelength band generated from the sample in the first reaction area and the optical signal of the second wavelength band (which differs from the first wavelength band) generated from the sample in the second reaction area 112. According to an embodiment of the disclosure, at least two of the plurality of light sources 210 may radiate excitation lights of different wavelength bands from each other. The excitation lights of different wavelength bands may excite different labels. The excited label may emit an emission light, which is an optical signal, and the different labels emit emission lights of different wavelength band. For example, emission light of a first wavelength band may excite a first label, and emission light of a second wavelength band may excite a second label. The number of the light sources 210 included in the optical module 200 is not particularly limited but, as an example, may be two or more or may be 100, 50, 40, 30, 20, 10 or less.

According to the disclosure, the optical module 200 may comprise a light source wheel 213. The light source wheel 213 receives the plurality of light sources 210. *The light source wheel 213 receives the light sources 210* may mean that the light sources 210 are attached or fixed to the light source wheel 213. The light source wheel 213 may be connected to a power transmission device, e.g., a motor, to rotate and may thus switch the light sources to illuminate each reaction area with light.

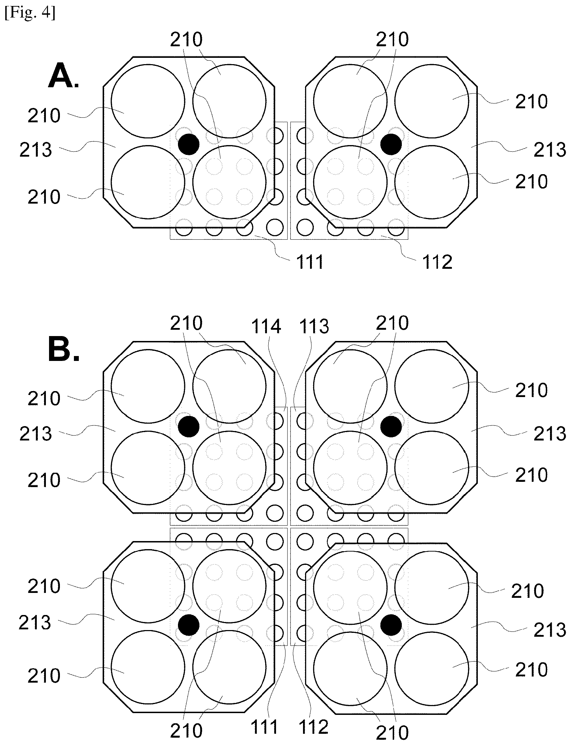

According to an embodiment of the disclosure, the wavelength band of the excitation light radiated to the first reaction area 111 and the wavelength band of the excitation light radiated to the second reaction area, each, may be independently determined. For example, referring to Figs. 2 and 4, a plurality of light sources may be configured to radiate excitation lights of different wavelength bands to the first reaction area, and another plurality of light sources may be separately configured to radiate excitation lights of different wavelength bands to the second reaction area. Specifically, as shown in Fig. 4A, the sample reaction module 100 according to the disclosure may comprise two reaction areas 111 and 112, and the optical module 200 comprises two light source wheels 213 each comprising a plurality of light sources 210 to allow each wavelength band of excitation light radiated to each reaction area to be independently determined. As another example, as shown in Fig. 4B, the sample reaction module 100 according to the disclosure may comprise four reaction areas 111, 112, 113, and 114. In this case, the optical module 200 may comprise four light source wheels 213 each comprising a plurality of light sources 210 for the four reaction areas.

The sample reaction module according to the disclosure may comprise four reaction areas. In this case, the optical module may comprise four light source wheels each comprising a plurality of light sources for the four reaction areas.

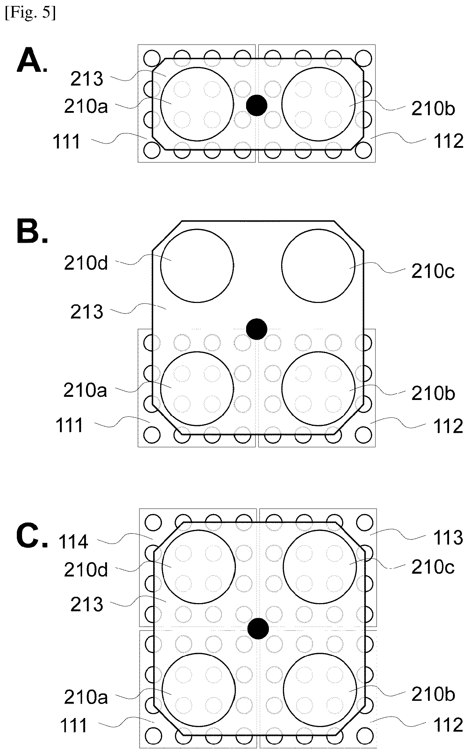

According to an embodiment of the disclosure, the wavelength band of the excitation light radiated to the first reaction area and the wavelength band of the excitation light radiated to the second reaction area may be synchronously changed. For example, as shown in Fig. 5, a plurality of light sources capable of radiating excitation lights of different wavelength bands may be connected to a moving member, e.g., the light source wheel 213 to be individually positioned on two or more reaction areas. For example, where the light source wheel 213 comprises two light sources for radiating light of different wavelength bands, the light sources may be positioned on the first reaction area 111 and the second reaction area 112 and may switch their positions (refer to Fig. 5A). Besides, as shown in Fig. 5B, the light source wheel 213 may comprise four light sources for radiating light of different wavelength bands and may synchronously radiate light of different wavelength bands to the first reaction area 111 and the second reaction area 112. In such a case, as the light source wheel 213 rotates, the light source 210a may be repositioned from the first reaction area 111 to the second reaction area 112, and another light source 210c may be positioned on the first reaction area 111.

As shown in Fig. 5C, the light source wheel 213 may comprise four light sources for radiating light of different wavelength bands And the four light sources may be respectively positioned on different reaction areas 111, 112, 113, and 114 to synchronously radiate light of different wavelength bands to the four reaction areas. In this case, as the light source wheel 213 rotates, the light source 210a may be moved from the first reaction area 111 to the second reaction area 112, the light source 210b may be moved from the second reaction area 112 to the third reaction area 113, the light source 210c may be moved from the third reaction area 113 to the fourth reaction area 114, and the light source 210d may be moved from the fourth reaction area 114 to the first reaction area 111. In the disclosure, such repositioning may be referred to as "cyclic movement." Such configuration may economically adjust the number of light sources used as compared with the number of the reaction areas.

Referring to Fig. 6, the light source 210 comprises a light emitting element 211. There may be provided a plurality of light emitting elements 211. The light emitting element may be a light emitting diode (LED). The light emitting element may be a light emitting element that selectively radiates light of a wavelength band that is to be emitted from the light source 210.

The light source 210 may comprise a filter 212. The filter 212 allows the reaction area to be illuminated with a light of a wavelength band intended by the light source 210. The filter 212 may be a filter that selectively transmits the light of the intended wavelength band among the lights generated from the light emitting element. According to an embodiment of the disclosure, the light source may comprise a plurality of filters, and the filters may be switched and used depending on the label used in the sample in the reaction area.

According to an embodiment of the disclosure, the optical module 200 comprises a plurality of light sources. At least two light sources of the plurality of light sources may produce excitation lights of different wavelength bands. According to an embodiment of the disclosure, the optical module 200 may selectively radiate excitation lights of two or more wavelength bands. The optical module 200 may comprise a plurality of light sources 210. The optical module 200 may comprise two or more light sources 210 that radiate light of different wavelength bands. In the optical module 200, each of light sources may be configured by selecting a light emitting element 211 that generates light of a specific wavelength band for the each of light sources. In the optical module 200, the filter 212 of each light source may be selected and configured as a filter that selectively transmits light of a different wavelength band for each light source. The plurality of light sources 210 that radiate light of different wavelength bands may be fastened to the light source wheel 213, and the light source wheel 213 may move the light sources 210 to allow light of different wavelength bands to be sequentially radiated to the reaction areas 111 and 112. By such a configuration, the optical module may selectively radiate excitation lights of different wavelength bands to one reaction area. Thus, the device according to the disclosure may detect a plurality of target nucleic acids that are detected by different labels in one sample.

According to the invention, the sample reaction module is configured so that each of the first reaction area and the second reaction area are capable of receiving a plurality of samples. According to the invention, the optical module comprises a plurality of light sources, and one of the plurality of light sources simultaneously radiates excitation lights to the plurality of samples in one reaction area. Thus, the optical module may detect optical signals from a plurality of samples more quickly than when one light source detects optical signals while sequentially radiating excitation lights to a plurality of samples.

Referring to Fig. 2, the optical module 200 according to the disclosure comprises a detector 220. The detector senses light. Specifically, the detector senses emission lights generated from samples. The detector may be, e.g., a CCD (Charge Coupled device), CMOS (Complementary Metal Oxide Semiconductor field effect transistor) or photodiode.

According to an embodiment of the disclosure, one detector may sense all of the emission lights generated from all the reaction areas. In this case, one detector is configured to separately detect different wavelength bands of emission lights generated from the plurality of reaction areas.

According to an embodiment of the disclosure, the optical module may comprise two or more detectors 220a and 220b as shown in Fig. 2. In such a case, each of the lights generated from the first reaction area 111 and the second reaction area 112 may be detected using an independent detector. According to an embodiment of the disclosure, the optical module comprises a plurality of detectors independently controlled, and one or more different detectors among the plurality of detectors may be assigned to each of the first reaction area and the second reaction area. Optical signals may be measured from the first sample set and second sample set by the one or more different detectors assigned.

According to an embodiment of the disclosure, two or more detectors may be used in one reaction area. In this case, two or more detectors may sense emission lights from one reaction area, with the reaction area separated.

The optical module is configured to be able to synchronously measure an optical signal of a first wavelength band from the sample in the first reaction area and an optical signal of a second wavelength band from the sample in the second reaction area, the second wavelength band different from the first wavelength band.

As shown in Fig. 7, according to an embodiment, the optical module may comprise a plurality of detectors 220a and 220b, and each of the plurality of detectors may be configured to sense the emission light generated from the sample positioned in a fixed and predetermined detection area 111 or 112 in the sample receiving module. The emission light refers to light that is generated from the sample and reaches the detector. Specifically, the emission light is light that is generated by a signal-generating means, e.g., the label in the sample. In the disclosure, the emission light corresponds to a signal or an optical signal.

The optical module 200 may be configured so that each of the plurality of detectors senses the emission light generated from the sample positioned in the respectively predetermined detection area. The detection area means an area where one detector detects an emission light in the sample reaction module. When the plurality of detectors each sense the emission light generated from the sample positioned in the predetermined detection area, this means that the detection area where each detector performs detection in the sample reaction module is previously determined. The samples positioned in the same detection area in the sample reaction module may be detected by the same detector, and the samples positioned in different detection areas may be detected by different detectors. In each detector, the detection area for sensing emission lights may be fixed. For example, a first detector may detect only emission lights generated from a first detection area, which is a fixed area in the sample reaction module but may not detect emission lights generated from other area.

According to an embodiment of the disclosure, one detection area may be included in one reaction area. In other words, the detection area may not be present over two or more reaction areas. It is preferable to measure the emission lights generated from different reaction areas by different detectors rather than measuring all of the lights synchronously generated from two or more reaction areas by one detector. For example, a first detector 220a may be configured to detect only emission lights generated from the first reaction area 111, but not emission lights generated from other reaction area, e.g., the second reaction area 112. Further, a second detector 220b may be configured to detect only emission lights generated from the second reaction area 112, but not emission lights generated from other area, e.g., the first reaction area 111.

According to an embodiment of the disclosure, one reaction area may comprise one or more detection areas. The number of detectors in charge of one reaction area may be determined depending on the shape and size of the reaction area and detection schemes, and the number of detectors may be one or two or more.

According to an embodiment of the disclosure, the optical module 200 may additionally comprise a filter 230 which may be positioned on an emission light path 270 to the detector. The filter may be a filter that selectively transmits the wavelength band of light, as the target for measurement, among the emission lights. According to an embodiment of the disclosure, the optical module may comprise a plurality of filters 230, and the filter positioned on the emission light path 270 may be replaced depending on the wavelength of the emission light to be measured. The replacement of the filter may be performed in such a manner that a plurality of filters 230 are attached to a filter wheel 235, and the filter wheel 235 is rotated as necessary.

Referring to Fig. 7, the optical module 200 may comprise a beam splitter 240. The beam splitter 240 reflects part of the excitation lights or emission lights, establishing an intended optical path. For example, as shown in Fig. 7, the excitation light generated from the light source 210 passes through the beam splitter 240 and reaches the sample received in the sample receiving space 115 of the reaction area 110, forming an excitation light path 260. The emission light emitted from the sample is reflected by the beam splitter 240 to the detector 220, forming an emission light path 270.

The optical module 200 may comprise a light blocking means 250. The light blocking means 250 may be configured so that the excitation light radiated from, e.g., the first reaction area 111 is not radiated to the second reaction area 112 or other reaction area. Further, the light blocking means 250 may be configured so that, when the excitation light is radiated from the light source 210a to the first reaction area 111, other lights than the radiated excitation light are not radiated to the first reaction area 111. The light blocking means 250 may be disposed between the adjacent light sources. Alternatively, the light blocking means 250 may be disposed between the adjacent detectors. According to an embodiment, the light blocking means 250 may be shaped as a rectangular or circular tube. According to an embodiment, the light blocking means 250 may be shaped as a combination of a plurality of tubes.

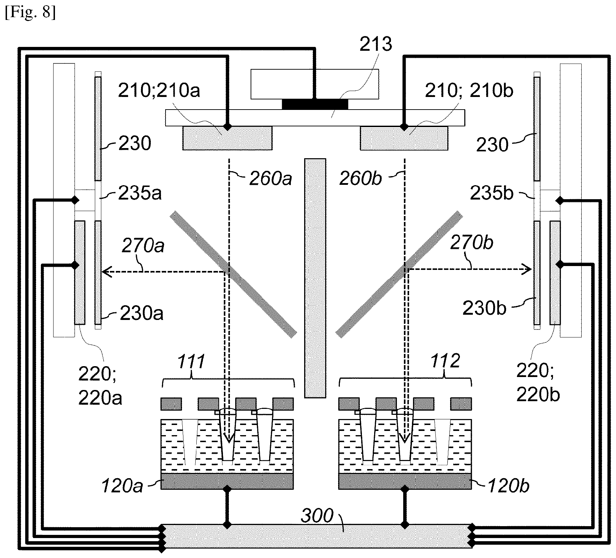

Referring to Fig. 8, the nucleic acid reaction detection device according to the disclosure comprises the controller 300. There may be provided one or more controllers 300 electrically connected with the sample reaction module 100 and the optical module 200. The controller may be, e.g., a computer, a micro-processor, or a programmable logic device.

The controller 300 controls each of the temperatures of the first reaction area 111 and the second reaction area 112 independently. Specifically, the controller 300 may be electrically connected with the heat transfer module 120a of the first reaction area and control the direction and magnitude of the current applied to the heat transfer module 120a of the first reaction area 111, thereby controlling the temperature of the first reaction area 111. Further, the controller 300 is electrically connected with the heat transfer module 120b of the second reaction area 112 and control the direction, magnitude, and time of the current applied to the heat transfer module 120b of the second reaction area 112 independently from the control of the temperature of the first reaction area 111, thereby controlling the temperature of the second reaction area 112. The controller 300 receives the protocol for the first sample set positioned in the first reaction area, controls the temperature of the first reaction area 111 according to the protocol for the first sample set, receives the protocol for the second sample set positioned in the second reaction area, and controls the temperature of the second reaction area 112 according to the protocol for the second sample set.

Further, according to an embodiment of the disclosure, the controller 300 is configured to control the optical signal measurement on the sample in the first reaction area and the optical signal measurement on the sample in the second reaction area independently. Specifically, the controller 300 may move the light source wheel 213 to move the first light source 210a or second light source 210b to a predetermined position so that the light of wavelength band capable of generating an optical signal from the sample in the first reaction area may be radiated to the first reaction area. Further, the controller 300 may supply current to the light source 210a so that the light source 210a is able to radiate the excitation light 260a to the sample. Further, the controller 300 may move the filter wheel 235a to position the filter 230a, which is able to selectively transmit the emission light 270a, on the emission light path 270a and supply current to the detector 220a so that the detector 220a is able to sense the emission light 270a generated from the sample by the excitation light 260a radiated from the light source 210a. To measure the optical signal from the sample in the second reaction area, the controller may move the light source wheel 213 and the filter wheel 230b and supply current to the light source 210b and the detector 220b as described above.

The controller 300 may operate the sample reaction module 100 and the optical module 200 of the nucleic acid reaction detection device so that the temperature control and optical signal measurement on each reaction area are performed in the order determined by the protocol.

The controller 300 may receive the protocol for each sample and according to the received protocol independently control the temperature of each reaction area of the sample reaction module and independently control the optical signal measurement on each reaction area of the optical module. For example, the controller 300 may operate the device to selectively perform optical signal measurement only on the sample in the first reaction area without measuring the optical signal for the sample in the second reaction area. Alternatively, the controller 300 may operate the device to selectively perform optical signal measurement only on the sample in the second reaction area without measuring the optical signal for the sample in the first reaction area. Or, the controller 300 may operate the device to synchronously perform optical signal measurement on the samples in the first reaction area and the second reaction area.

According to an embodiment of the disclosure, the controller may receive distinct protocols for the first reaction area and the second reaction area, control temperatures of the first reaction area and the second reaction area according to the received protocols independently, and operate the nucleic acid reaction detection device to perform an operation comprising at least one of the steps of, based on the received protocols for all the reaction areas:

(i) measuring an optical signal for a sample in one reaction area of the first reaction area and the second reaction area without measuring an optical signal for a sample in the other reaction area; and (ii) synchronously measuring an optical signal of a first wavelength band from a sample in the first reaction area and an optical signal of a second wavelength band from a sample in the second reaction area, wherein the second wavelength band is different from the first wavelength band.

Reaction is performed on the sample positioned in the first reaction area and the sample positioned in the second reaction area by protocols independent from each other, and the first reaction area and the second reaction area may individually or simultaneously reach the light detection time. When it becomes time to detecting optical signal only for one of the first reaction area and the second reaction area, the controller 300 operates to perform the operation of measuring the optical signal for the sample in one reaction area of the first reaction area and the second reaction area without measuring the optical signal for the sample in the other reaction area. Further, when it becomes time to detecting optical signals for both the first reaction area and the second reaction area, the controller 300 operates to perform the operation of measuring the optical signal for the sample in each reaction area. At this time, the wavelength bands of the optical signals measured from the first reaction area and the second reaction area may differ from each other.

According to an embodiment of the disclosure, the controller may be configured to control the sample reaction module and optical module to be able to selectively perform optical signal measurement on one area of the first reaction area and the second reaction area under the condition where the temperatures of the first reaction area and the second reaction area differ from each other. For example, referring to Fig. 8, the controller 300 may control the light source 210a, filter wheel 230a, and detector 220a for the first reaction area to measure the optical signal for the first reaction area while simultaneously controlling the heat transfer module 120 of the second reaction area 112 to allow a nucleic acid reaction to occur in the second reaction area.

Further, the controller may operate the device to be able to detect a plurality of target nucleic acids in one sample. Specifically, the controller may allow optical signals of two or more different wavelength bands to be measured sequentially for one reaction area. According to an embodiment of the disclosure, the controller may be configured to control the sample reaction module and optical module to be able to sequentially measure optical signals of two or more different wavelength bands for one area of the first reaction area and the second reaction area under the condition where the temperatures of the first reaction area and the second reaction area differ from each other.

According to an embodiment, referring to Fig. 8, at a first time, the controller may move the first light source 210a to the position where the excitation light may be radiated to the first reaction area 111 and applies current to allow the first light source 210a to radiate an excitation light. Then, the controller may drive the filter wheel 235a to position the filter, which is able to selectively transmit the emission light generated from the sample by the excitation light from the first light source 210a, on the excitation light path 270a and apply current to the detector 220a, thereby measuring the optical signal from the first reaction area 111. After the optical signal measurement using the first light source 210a is finished, the controller drives the light source wheel 213 to move the second light source 210b to the position where it may radiate the excitation light to the first reaction area 111 at a second time and applies current to allow the second light source 210b to radiate the excitation light. Then, the controller may drive the filter wheel 235a to position the filter, which is able to measure the emission light generated from the sample by the excitation light from the second light source 210b, on the excitation light path 270a and apply current to the detector 220a, thereby measuring the optical signal from the first reaction area 111. While the above-described optical signal measurement is carried out on the first reaction area 111, the controller 300 may change the temperature of the second reaction area 112 and perform nucleic acid reaction.

By the above-described operations of the controller 300, the nucleic acid reaction detection device 10 may detect each of a plurality of targets for each of a plurality of sample sets where optical signal detection on the plurality of targets and nucleic acid reaction are performed according to different protocols.

While embodiments of the disclosure have been described above, it will be easily appreciated by one of ordinary skill in the art that the disclosure is not limited thereto. Thus, the scope of the invention is defined by the appended claims.

### [CROSS-REFERENCE TO RELATED APPLICATIONS]

This application claims priority from Korean Patent Application No. 10-2019-0078284, filed on June 28, 2019, and Korean Patent Application No. 10-2019-0064472, filed on May 31, 2019, Korean Patent Application No. 10-2019-0162094, filed on December 6, 2019, in the Korean Intellectual Property Office.

## Claims

1. A method for detecting a target nucleic acid in a sample, the method comprising the steps of:
(a) positioning a first sample set in a first reaction area (111) of a nucleic acid reaction detection device (10) and a second sample set in a second reaction area (112) of the nucleic acid reaction detection device (10) and performing a nucleic acid reaction; wherein
the nucleic acid reaction detection device comprises a sample reaction module (100) comprising the first reaction area (111) and the second reaction area (112) thermally independent from each other, an optical module (200) for detecting nucleic acid reaction of samples in the first reaction area and the second reaction area, and a controller (300);
wherein temperatures of the first reaction area and the second reaction area are controlled according to different protocols;
wherein the sample reaction module is configured so that each of the first reaction area and the second reaction area is capable of receiving a plurality of samples; and wherein the optical module comprises a plurality of light sources (210, 210a, 210b, 210c, 210d), and one of the plurality of light sources simultaneously radiates excitation light to the plurality of samples in one reaction area;
(b) measuring optical signals from the first sample set and the second sample set; wherein the optical signals from the first sample set and the second sample set are measured synchronously; wherein the wavelength bands of the optical signals synchronously measured from the first sample set and the second sample set are different from each other; and
(c) detecting a target nucleic acid in a sample included in the first sample set and the second sample set by analyzing the measured optical signals.

2. The method according to claim 1, wherein the step of measuring the optical signals further comprises a step of measuring an optical signal from one of the first sample set and the second sample set without measuring an optical signal from the other of the first sample set and the second sample set.

3. The method according to claim 1, wherein the measurement of an optical signal for the first sample set is performed by sequentially measuring optical signals of two or more different wavelength bands for the first sample set.

4. The method according to claim 1, wherein the first sample set comprises a plurality of samples, and wherein measuring an optical signal from the first sample set positioned in the first reaction area is simultaneously measuring optical signals from two or more samples included in the first sample set.

5. The method according to claim 1, wherein the controller operates the nucleic acid reaction detection device to perform an operation further comprising a step of:
measuring an optical signal for one sample set of the first sample set and the second sample set without measuring an optical signal for the other sample set.

6. The method according to claim 1, wherein at least two of the plurality of light sources produce excitation lights of different wavelength bands from each other.

7. The method according to claim 1, wherein the optical module (200) comprises a plurality of detectors (220, 220a, 220b), and wherein each of the plurality of detectors detects light emitted from a sample in a predetermined and fixed area of the sample reaction module.

8. The method according to claim 1, wherein the optical module (200) comprises a plurality of detectors (220, 220a, 220b) independently controlled,
wherein one or more different detectors among the plurality of detectors are assigned to each of the first reaction area (111) and the second reaction area (112),
and wherein optical signals are detected from the first sample set and the second sample set by the one or more different detectors assigned respectively.

9. A nucleic acid reaction detection device (10), comprising:
a sample reaction module (100) comprising a first reaction area (111) and a second reaction area (112) thermally independent from each other;
an optical module (200) for detecting nucleic acid reaction of samples in the sample reaction module; and
one or more controllers (300) electrically connected with the sample reaction module and the optical module, wherein
the optical module is configured to synchronously measure an optical signal of a first wavelength band from a sample in the first reaction area and an optical signal of a second wavelength band from a sample in the second reaction area, wherein the second wavelength band is different from the first wavelength band,
wherein the sample reaction module is configured so that each of the first reaction area and the second reaction area is capable of receiving a plurality of samples;
wherein the optical module comprises a plurality of light sources (210, 210a, 210b, 210c, 210d), and one of the plurality of light sources simultaneously radiates excitation light to the plurality of samples in one reaction area;
and wherein the controller is configured to control temperatures of the first reaction area and the second reaction area independently and control optical signal measurement of a sample in the first reaction area and optical signal measurement of a sample in the second reaction area independently.

10. The nucleic acid reaction detection device according to claim 9, wherein the controller receives distinct protocols for the first reaction area and the second reaction area; controls temperatures of the first reaction area and the second reaction area independently according to the received protocols; and operates the nucleic acid reaction detection device to perform an operation comprising at least one of the steps of, based on the received protocols for all the reaction areas:
(i) measuring an optical signal for a sample in one reaction area of the first reaction area and the second reaction area without measuring an optical signal for a sample in the other reaction area; and
(ii) synchronously measuring an optical signal of a first wavelength band from a sample in the first reaction area and an optical signal of a second wavelength band from a sample in the second reaction area, wherein the second wavelength band is different from the first wavelength band.

11. The nucleic acid reaction detection device according to claim 9, wherein the optical module (200) comprises a plurality of detectors (220, 220a, 220b), and wherein each of the plurality of detectors detects light emitted from a sample in a predetermined and fixed area of the sample reaction (100).

12. The nucleic acid reaction detection device according to claim 9, wherein at least two of the plurality of light sources (210, 210a, 210b, 210c, 210d) produce excitation lights of different wavelength bands from each other.

## Patentansprüche

1. Verfahren zum Detektieren einer Ziel-Nukleinsäure in einer Probe, wobei das Verfahren die folgenden Schritte umfasst:
(a) Positionieren eines ersten Probensatzes in einem ersten Reaktionsbereich (111) einer Nukleinsäurereaktions-Detektionsvorrichtung (10) und eines zweiten Probensatzes in einem zweiten Reaktionsbereich (112) der Nukleinsäurereaktions-Detektionsvorrichtung (10) und Durchführen einer Nukleinsäurereaktion; wobei die Nukleinsäurereaktions-Detektionsvorrichtung ein Probenreaktionsmodul (100), das den ersten Reaktionsbereich (111) und den zweiten Reaktionsbereich (112) umfasst, die thermisch unabhängig voneinander sind, ein optisches Modul (200) zum Detektieren einer Nukleinsäurereaktion der Proben in dem ersten Reaktionsbereich und dem zweiten Reaktionsbereich und eine Steuerung (300) umfasst;
wobei die Temperaturen des ersten Reaktionsbereichs und des zweiten Reaktionsbereichs gemäß unterschiedlicher Protokolle gesteuert werden;
wobei das Probenreaktionsmodul so konfiguriert ist, dass jeder des ersten Reaktionsbereichs und des zweiten Reaktionsbereichs in der Lage ist, eine Vielzahl von Proben aufzunehmen;
und wobei das optische Modul eine Vielzahl von Lichtquellen (210, 210a, 210b, 210c, 210d) umfasst, und eine der Vielzahl von Lichtquellen gleichzeitig Anregungslicht auf die Vielzahl von Proben in einem Reaktionsbereich strahlt;
(b) Messen optischer Signale von dem ersten Probensatz und dem zweiten Probensatz; wobei die optischen Signale von dem ersten Probensatz und dem zweiten Probensatz zeitgleich gemessen werden; wobei die Wellenlängenbänder der optischen Signale, die zeitgleich von dem ersten Probensatz und dem zweiten Probensatz gemessen werden, sich voneinander unterscheiden; und
(c) Detektieren einer Ziel-Nukleinsäure in einer Probe, die in dem ersten Probensatz und dem zweiten Probensatz enthalten ist, durch Analysieren der gemessenen optischen Signale.

2. Verfahren nach Anspruch 1, wobei der Schritt des Messens der optischen Signale ferner einen Schritt des Messens eines optischen Signals von einem des ersten Probensatzes und des zweiten Probensatzes umfasst, ohne dass ein optischen Signal von dem anderen des ersten Probensatzes und des zweiten Probensatzes gemessen wird.

3. Verfahren nach Anspruch 1, wobei die Messung eines optischen Signals für den ersten Probensatz durch das sequenzielle Messen optischer Signale von zwei oder mehreren unterschiedlichen Wellenlängenbändern für den ersten Probensatz durchgeführt wird.

4. Verfahren nach Anspruch 1, wobei der erste Probensatz eine Vielzahl von Proben umfasst, und wobei das Messen eines optischen Signals von dem ersten Probensatz, der im ersten Reaktionsbereich positioniert ist, gleichzeitig optische Signale von zwei oder mehreren Proben misst, die im ersten Probensatz enthalten sind.

5. Verfahren nach Anspruch 1, wobei die Steuerung die Nukleinsäurereaktions-Detektionsvorrichtung betreibt, um eine Funktion durchzuführen, die ferner den folgenden Schritt umfasst:
Messen eines optischen Signals für einen Probensatz des ersten Probensatzes und des zweiten Probensatzes, ohne dass ein optisches Signal für den anderen Probensatz gemessen wird.

6. Verfahren nach Anspruch 1, wobei mindestens zwei aus der Vielzahl von Lichtquellen Anregungslichter mit voneinander verschiedenen Wellenlängenbändern erzeugen.

7. Verfahren nach Anspruch 1, wobei das optische Modul (200) eine Vielzahl von Detektoren (220, 220a, 220b) umfasst, und wobei jeder der Vielzahl von Detektoren Licht detektiert, das von einer Probe in einem vorbestimmten und festen Bereich des Probenreaktionsmoduls emittiert wird.

8. Verfahren nach Anspruch 1, wobei das optische Modul (200) eine Vielzahl von Detektoren (220, 220a, 220b) umfasst, die unabhängig voneinander gesteuert werden,
wobei ein oder mehrere verschiedene Detektoren aus der Vielzahl von Detektoren jeweils dem ersten Reaktionsbereich (111) und dem zweiten Reaktionsbereich (112) zugeordnet werden,
und wobei optische Signale von dem ersten Probensatz und dem zweiten Probensatz durch den einen oder die mehreren verschiedenen, jeweils zugeordneten Detektoren detektiert werden.

9. Nukleinsäurereaktions-Detektionsvorrichtung (10), die Folgendes umfasst:
ein Probenreaktionsmodul (100), das einen ersten Reaktionsbereich (111) und einen zweiten Reaktionsbereich (112) umfasst, die thermisch unabhängig voneinander sind;
ein optisches Modul (200) zum Detektieren einer Nukleinsäurereaktion der Proben in dem Probenreaktionsmodul; und
eine oder mehrere Steuerungen (300), die mit dem Probenreaktionsmodul und dem optischen Modul elektrisch verbunden sind, wobei
das optische Modul dazu konfiguriert ist, ein optisches Signal eines ersten Wellenlängenbands aus einer Probe in dem ersten Reaktionsbereich und ein optisches Signal eines zweiten Wellenlängenbands aus einer Probe in dem zweiten Reaktionsbereich zeitgleich zu messen, wobei das zweite Wellenlängenband sich von dem ersten Wellenlängenband unterscheidet,
wobei das Probenreaktionsmodul so konfiguriert ist, dass jeder des ersten Reaktionsbereichs und des zweiten Reaktionsbereichs in der Lage ist, eine Vielzahl von Proben aufzunehmen;
wobei das optische Modul eine Vielzahl von Lichtquellen (210, 210a, 210b, 210c, 210d) umfasst, und eine der Vielzahl von Lichtquellen gleichzeitig Anregungslicht auf die Vielzahl von Proben in einem Reaktionsbereich strahlt;
und wobei die Steuerung dazu konfiguriert ist, die Temperaturen des ersten Reaktionsbereichs und des zweiten Reaktionsbereichs unabhängig voneinander zu steuern und eine optische Signalmessung einer Probe in dem ersten Reaktionsbereich und eine optische Signalmessung einer Probe in dem zweiten Reaktionsbereich unabhängig voneinander zu steuern.

10. Nukleinsäurereaktions-Detektionsvorrichtung nach Anspruch 9, wobei die Steuerung unterschiedliche Protokolle für den ersten Reaktionsbereich und den zweiten Reaktionsbereich empfängt; die Temperaturen des ersten Reaktionsbereichs und des zweiten Reaktionsbereichs unabhängig voneinander gemäß den empfangenen Protokollen steuert; und die Nukleinsäurereaktions-Detektionsvorrichtung betreibt, um eine Funktion durchzuführen, die basierend auf den empfangenen Protokollen für alle Reaktionsbereiche mindestens einen der folgenden Schritte umfasst:
(i) Messen eines optischen Signals für eine Probe in einem Reaktionsbereich des ersten Reaktionsbereichs und des zweiten Reaktionsbereichs, ohne dass ein optisches Signal für eine Probe in dem anderen Reaktionsbereich gemessen wird; und
(ii) zeitgleiches Messen eines optischen Signals eines ersten Wellenlängenbands von einer Probe in dem ersten Reaktionsbereich und eines optischen Signals eines zweiten Wellenlängenbands von einer Probe in dem zweiten Reaktionsbereich, wobei das zweite Wellenlängenband sich von dem ersten Wellenlängenband unterscheidet.

11. Nukleinsäurereaktions-Detektionsvorrichtung nach Anspruch 9, wobei das optische Modul (200) eine Vielzahl von Detektoren (220, 220a, 220b) umfasst, und wobei jeder der Vielzahl von Detektoren Licht detektiert, das von einer Probe in einem vorbestimmten und festen Bereich der Probenreaktion (100) emittiert wird.

12. Nukleinsäurereaktions-Detektionsvorrichtung nach Anspruch 9, wobei mindestens zwei aus der Vielzahl von Lichtquellen (210, 210a, 210b, 210c, 210d) Anregungslichter mit voneinander verschiedenen Wellenlängenbändern erzeugen.

## Revendications

1. Procédé de détection d'un acide nucléique cible dans un échantillon, le procédé comprenant les étapes suivantes :
(a) le positionnement d'un premier ensemble d'échantillons dans une première zone (111) de réaction d'un dispositif (10) de détection de réaction d'acide nucléique et d'un deuxième ensemble d'échantillons dans une deuxième zone (112) de réaction du dispositif (10) de détection de réaction d'acide nucléique et la mise en oeuvre d'une réaction d'acide nucléique ; dans lequel le dispositif de détection de réaction d'acide nucléique comprend un module (100) de réaction d'échantillon comprenant la première zone (111) de réaction et la deuxième zone (112) de réaction thermiquement indépendantes l'une de l'autre, un module optique (200) pour détecter une réaction d'acide nucléique d'échantillons dans la première zone de réaction et la deuxième zone de réaction, et un dispositif de commande (300) ; dans lequel les températures de la première zone de réaction et de la deuxième zone de réaction sont régulées conformément à des protocoles différents ; dans lequel le module de réaction d'échantillon est configuré de sorte que chacune parmi la première zone de réaction et la deuxième zone de réaction est capable de recevoir une pluralité d'échantillons ; et dans lequel le module optique comprend une pluralité de sources de lumière (210, 210a, 210b, 210c, 210d), et une de la pluralité de sources de lumière irradie simultanément une lumière d'excitation sur la pluralité d'échantillons dans une zone de réaction ;
(b) la mesure de signaux optiques provenant du premier ensemble d'échantillons et du deuxième ensemble d'échantillons ; dans lequel les signaux optiques provenant du premier ensemble d'échantillons et du deuxième ensemble d'échantillons sont mesurés de manière synchrone ; dans lequel les bandes de longueur d'onde des signaux optiques mesurés de manière synchrone à partir du premier ensemble d'échantillons et du deuxième ensemble d'échantillons sont différentes les unes des autres ; et
(c) la détection d'un acide nucléique cible dans un échantillon inclus dans le premier ensemble d'échantillons et le deuxième ensemble d'échantillons en analysant les signaux optiques mesurés.

2. Procédé selon la revendication 1, dans lequel l'étape de mesure des signaux optiques comprend en outre une étape de mesure d'un signal optique provenant de l'un parmi le premier ensemble d'échantillons et le deuxième ensemble d'échantillons sans mesurer un signal optique provenant de l'autre parmi le premier ensemble d'échantillons et le deuxième ensemble d'échantillons.

3. Procédé selon la revendication 1, dans lequel la mesure d'un signal optique pour le premier ensemble d'échantillons est effectuée en mesurant séquentiellement des signaux optiques de deux bandes de longueurs d'onde différentes ou plus pour le premier ensemble d'échantillons.

4. Procédé selon la revendication 1, dans lequel le premier ensemble d'échantillons comprend une pluralité d'échantillons, et dans lequel la mesure d'un signal optique provenant du premier ensemble d'échantillons positionné dans la première zone de réaction mesure simultanément des signaux optiques provenant de deux échantillons ou plus inclus dans le premier ensemble d'échantillons.

5. Procédé selon la revendication 1, dans lequel le dispositif de commande fait fonctionner le dispositif de détection de réaction d'acide nucléique pour effectuer une opération comprenant en outre une étape suivante :
la mesure d'un signal optique pour un ensemble d'échantillons du premier ensemble d'échantillons et du deuxième ensemble d'échantillons sans mesurer un signal optique pour l'autre ensemble d'échantillons.

6. Procédé selon la revendication 1, dans lequel au moins deux de la pluralité de sources de lumière produisent des lumières d'excitation de bandes de longueur d'onde différentes les unes des autres.

7. Procédé selon la revendication 1, dans lequel le module optique (200) comprend une pluralité de détecteurs (220, 220a, 200b), et dans lequel chacun de la pluralité de détecteurs détecte une lumière émise par un échantillon dans une zone prédéterminée et fixe du module de réaction d'échantillon.

8. Procédé selon la revendication 1, dans lequel le module optique (200) comprend une pluralité de détecteurs (220, 220a, 200b) commandés indépendamment, dans lequel un ou plusieurs détecteurs différents parmi la pluralité de détecteurs sont attribués à chacune parmi la première zone (111) de réaction et la deuxième zone (112) de réaction, et dans lequel des signaux optiques sont détectés à partir du premier ensemble d'échantillons et du deuxième ensemble d'échantillons par les un ou plusieurs détecteurs différents attribués respectivement.

9. Dispositif (10) de détection de réaction d'acide nucléique, comprenant :
un module (100) de réaction d'échantillon comprenant une première zone (111) de réaction et une deuxième zone (112) de réaction thermiquement indépendantes l'une de l'autre ;
un module optique (200) pour détecter une réaction d'acide nucléique d'échantillons dans le module de réaction d'échantillons ; et
un ou plusieurs dispositifs de commande (300) connectés électriquement au module de réaction d'échantillon et au module optique, dans lequel
le module optique est configuré pour mesurer de manière synchrone un signal optique d'une première bande de longueur d'onde provenant d'un échantillon dans la première zone de réaction et un signal optique d'une deuxième bande de longueur d'onde provenant d'un échantillon dans la deuxième zone de réaction, dans lequel la deuxième bande de longueur d'onde est différente de la première bande de longueur d'onde,
dans lequel le module de réaction d'échantillon est configuré de sorte que chacune parmi la première zone de réaction et la deuxième zone de réaction est capable de recevoir une pluralité d'échantillons ;
dans lequel le module optique comprend une pluralité de sources de lumière (210, 210a, 210b, 210c, 210d), et
une de la pluralité de sources de lumière irradie simultanément une lumière d'excitation sur la pluralité d'échantillons dans une zone de réaction ;
et dans lequel le dispositif de commande est configuré pour réguler les températures de la première zone de réaction et de la deuxième zone de réaction de manière indépendante et commander la mesure de signal optique d'un échantillon dans la première zone de réaction et la mesure de signal optique d'un échantillon dans la deuxième zone de réaction de manière indépendante.

10. Dispositif de détection de réaction d'acide nucléique selon la revendication 9, dans lequel le dispositif de commande reçoit des protocoles distincts pour la première zone de réaction et la deuxième zone de réaction ; régule les températures de la première zone de réaction et de la deuxième zone de réaction de manière indépendante conformément aux protocoles reçus ; et fait fonctionner le dispositif de détection de réaction d'acide nucléique pour effectuer une opération comprenant au moins l'une des étapes suivantes, sur la base des protocoles reçus pour toutes les zones de réaction :
(i) la mesure d'un signal optique pour un échantillon dans une zone de réaction parmi la première zone de réaction et la deuxième zone de réaction sans mesurer un signal optique pour un échantillon dans l'autre zone de réaction ; et
(ii) la mesure de manière synchrone d'un signal optique d'une première bande de longueur d'onde provenant d'un échantillon dans la première zone de réaction et d'un signal optique d'une deuxième bande de longueur d'onde provenant d'un échantillon dans la deuxième zone de réaction, dans lequel la deuxième bande de longueur d'onde est différente de la première bande de longueur d'onde.

11. Dispositif de détection de réaction d'acide nucléique selon la revendication 9, dans lequel le module optique (200) comprend une pluralité de détecteurs (200, 200a, 200b), et dans lequel chacun de la pluralité de détecteurs détecte une lumière émise par un échantillon dans une zone prédéterminée et fixe de la réaction d'échantillon (100).

12. Dispositif de détection de réaction d'acide nucléique selon la revendication 9, dans lequel au moins deux de la pluralité de sources de lumière (210, 210a, 210b, 210c, 210d) produisent des lumières d'excitation de bandes de longueurs d'onde différentes les unes des autres.
